# EUROPEAN PATENT SPECIFICATION

(11) **EP 1 278 487 B1**
(45) Date of publication and mention of the grant of the patent: **13.09.2006**
(21) Application number: 01930931.9
(22) Date of filing: 30.04.2001
(51) Int. Cl.: A61F 2/44

(54) **ROTATING, LOCKING INTERVERTEBRAL DISK STABILIZER**
DREH- UND VERRIEGELBARER BANDSCHEIBENSTABILISATOR UND EINBRINGVORRICHTUNG
DISQUE STABILISATEUR INTERVERTEBRAL TOURNANT DE VERROUILLAGE

(30) Priority: 28.04.2000 US 561483
(43) Date of publication of application: 29.01.2003
(73) Proprietor: Perumala Corporation, Brownsville, TX 78520 (US)
(72) Inventor: PISHARODI, Madhavan, Brownsville, TX 78520 (US)
(74) Representative: Lockey, Robert Alexander
(86) International application number: PCT/US2001/013824
(87) International publication number: WO 2001/082844

(56) References cited:
- EP-A- 0 260 044
- WO-A-00/40179
- WO-A-95/08306
- WO-A-96/40016
- WO-A-99/60957
- US-A- 5 658 336
- US-A- 5 716 415
- US-A- 5 893 890

## Description

The present invention relates to an intervertebral disk stabilizing implant for stabilizing two adjacent vertebrae. More specifically, the present invention relates to rectangularly-shaped disk implants which are expanded in the middle portion and are used for spinal fusion.

Treatment of a herniated disk in the neck and in the lumbar region continues to be a challenging field of medicine. The classical treatment for a ruptured disk is diskectomy, i.e., removal of the disk from between the vertebrae. In this process, all or a portion of the intervertebral disk is removed, leaving a defect which continues to bother the patients throughout the rest of their lives. An additional procedure is to replace the disk space with a bone graft, usually bone chips cut from the patient's iliac crest, bringing about fusion of the vertebrae above and below the disk and eliminating the empty space between the vertebrae.

Diskectomy with fusion is not ideal because the replaced bone does not have the function of the cartilaginous tissue of the disk, i.e. no cushioning effect, and has complications because of several factors. First, conventional bone plugs used to pack the disk space do not conform to the space of the disk because the disk bulges maximally in the center. The disk space is wider in the middle and narrower at its anterior and posterior ends. For this reason, the various bone plugs which are currently available commercially have only four contact points, i.e. at the front and back of the disk space. Secondly, access to the disk is from the side of the dorsal spine of the adjacent vertebrae, leaving a space that is "off-center" relative to the bodies of the adjacent vertebrae such that the stability of the implant is even more problematical than might be apparent from the limited contact resulting from the shape of the intervertebral space. Another complication is the possibility of infection or other conditions that may require removal of the implant. Also, if the bone pieces do not fuse, they may eventually extrude out of the disk space, causing pressure on the nerve roots.

Various prosthetic disk plugs, or implants, are disclosed in the art, but all are characterized by limitations of not conforming to the shape of the disk space, lack of stability when inserted off-center, inability to be removed, or other disadvantages. For instance, U.S. Patent No. 4,863,476 (and its European counterpart, EP-A-0260044) describes an elongated body divided longitudinally into two portions having a cam device movable therebetween for increasing the space between the two body portions once inserted into the disk space. However, that device is generally cylindrical in shape such that the only contact points between the device and the vertebral bodies are at the front and back of the disk space, creating increased likelihood of instability and generally rendering that device unsuitable for use after partial diskectomy. The art also discloses intervertebral disk prostheses (e.g. U.S. Patent Nos. 3,867,728, 4,309,777, 4,863,477 and 4,932,969 and French Patent Application No. 8816184) which may have more general contact with the adjacent disks, but which are not intended for use in fusion of the disks. The art also includes spinal joint prostheses such as is described in U.S. Patent No. 4,759,769, which is again not indicated for use when fusion is the preferred surgical intervention.

Published PCT Application WO96/40016 discloses an intervertebral disk stabilizer having a lock that engages the end of the implant There are, however, indications for use of an intervertebral stabilizer in which an increased likelihood of successful fusion can be obtained

There is, therefore, a need for a device capable of stablizing the vertebrae adjacent an intervertebral disk, but which is also removable, for use in spinal fusion. There is also a need for a method of implanting such a stabilizer.

Because of its desirable properties when used for fusion of adjacent vertebrae, there is also a need for an implant having these advantages but which also has a construction, or shape, that lends itself to being fabricated from bone.

U.S. 5,658,336 discloses a rotating, rocking, middle-expanded intervertebral disk stabilizer. The stabilizer incorporates an implant which has first and second sides defining the height of the implant and third and fourth sides which define the width of the implant. The sides which define the width of the implant are arched from one end to the other. The implant is a bi-planar, high-convex implant.

WO00/40179 discloses a flexible implant using a partially demineralised bone. The implant is useful for creating bony fusion, particularly in intervertebral spinal fusion. The device is formed of bone and has an at least partially demineralised portion between two rigid bone portions creating an area of flexibility. The bone portions may move, in a manner similar to that of a pair of tongs, to enable the device to be located in position between two vertebrae.

According to this invention there is provided an apparatus for use in fusing adjacent vertebrae, the apparatus comprising an elongate implant to be located between adjacent vertebrae, the implant being of elongate form and having two opposed sides which define the width W of the implant, each of the two opposed sides being of a side edge of arched form, wherein the opposed edges are such that an end part of the implant is flared so that a portion of the implant between the flared end part and the remainder of the implant is of lesser width that the width of the flare.

Preferably the arched edges and flare form an upwardly open recess in the top part of the implant and a downwardly open recess in the bottom part of the implant, the recesses being smoothly curved recesses.

Conveniently the recesses are substantially symmetrical about a horizontal plane dividing the upper and lower parts of the implant.

Advantageously the flared end part of the implant is constituted by at least one block which is movably mounted on the rest of the implant for changing the width of the implant at the flared end part thereof.

Preferably there are two said blocks, each slidably mounted to the rest of the implant.

Conveniently each of said blocks is threadably engaged with a rotatable spindle, the spindle being mounted on the rest of the implant, means being provided to rotate the spindle.

Advantageously the implant has two opposed substantially planar faces.

Conveniently key-ways are formed in the planar faces and extend axially of the implant.

Advantageously each key-way is provided with a funnel shaped terminal portion, the funnel shaped portion converging to a width approximately equal to the width of the main part of the key-way.

Preferably the apparatus further comprises an applicator having prongs adapted to co-operate with said key-ways.

In order that the invention may be more readily understood and so that further aspects thereof may be appreciated, a disk stabilizer devised by the inventor of the present invention and embodiments of the invention will now be described, by way of example, with reference to the accompanying drawings in which:
FIGURE 1 is a lateral view of a portion of a human spinal column having a vertebral disk stabilizer inserted therein and having a portion of the bodies of the vertebrae adjacent the implant shown cut away and/or in shadow lines to show the engagement of the vertebral bodies by the vertebral disk stabilizer,
FIGURE 2 is an enlarged, perspective, partially schematic view of the vertebral disk stabilizer of Figure 1 in place between two adjacent vertebral bodies.
FIGURE 3 is a perspective view of the implant of the vertebral disk stabilizer of Figures 1 and 2 and an applicator to which the implant is mounted for placing the implant in the position shown in Figure 1,
FIGURE 4 is a perspective view of a human femur cut in cross-section to show a method fabricating an implant,
FIGURE 5A is a partially schematic lateral elevational view of a portion of the human skeleton comprising the hip joint, the ilium and a portion of the femur to show a method of fabricating the locking piece of the vertebral disk stabilizer,
FIGURE 5B is a perspective view of a portion of the iliac crest after cutting along the shadow lines in Figure 5A,
FIGURE 6A, 6B and 6C are elevational views of an embodiment of an implant constructed in accordance with the teachings of the present invention,
FIGURE 7 is an elevational view of a second embodiment of an implant constructed in accordance with the teachings of the present invention, and
FIGURE 8 is a perspective view of a third embodiment of an implant constructed in accordance with the teachings of the present invention.

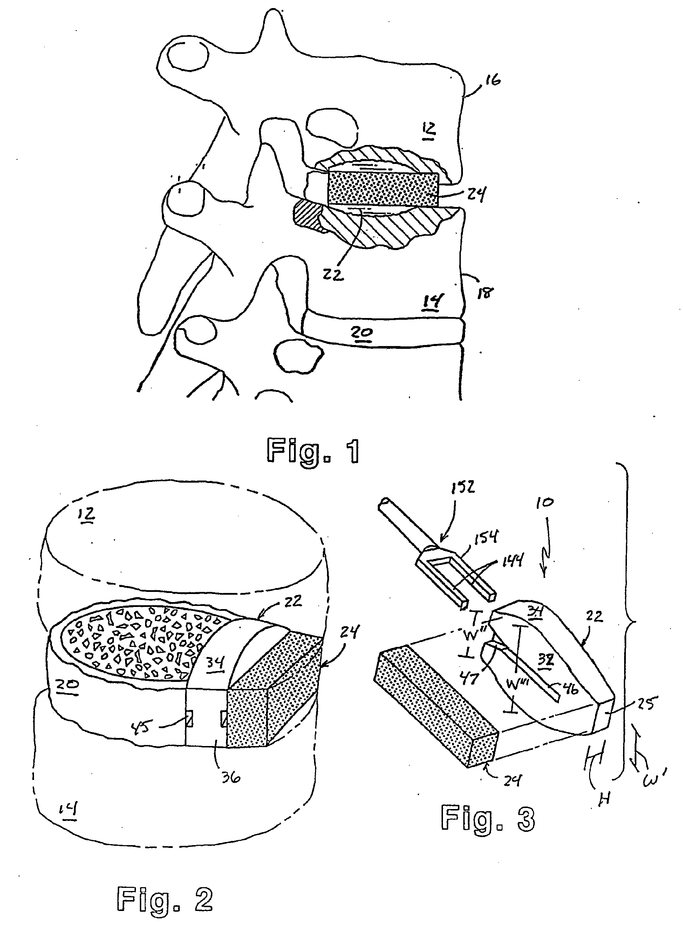

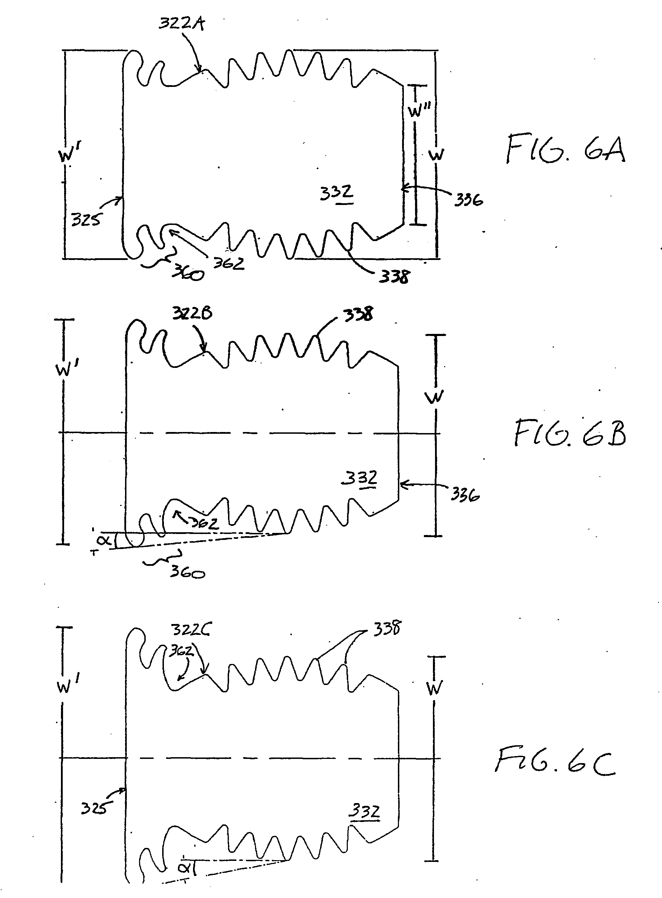

Referring now to the figures, a first embodiment of a disk stabilizer devised by the inventor of the present invention is shown implanted in a human will be described with reference to Figures 1 to 5, as this description will facilitate an understanding of the invention, as described with reference to Figures 6 to 8. The first embodiment of a disk stabiliser. spinal column in Fig. 1. The vertebral disk stabilizer, indicated generally at reference numeral 10, is implanted between the bodies 12 and 14 of two adjacent vertebrae 16 and 18, respectively, in the disk space (not numbered) from which a portion of the intervertebral disk 20 is removed, i.e. by simple diskectomy and small laminotomy.

Referring now also to Figs. 2 and 3, the first embodiment of the vertebral disk stabilizer 10 shown is comprised of an elongate implant 22 and locking piece 24. In more detail, implant 22 is comprised of first and second sides 32 and third and fourth sides 34 providing a substantially rectangularly shaped cross-section. The height H of the rectangular shaped cross-section is defined by first and second sides 32 and the width W is defined by the third and fourth sides 34 and, as is apparent by comparison of H and W, the height of H of implant 22 is less than the width W. As will be explained below, H is minimized to facilitate insertion of the second end 36 into, and positioning of implant 22 in, the disk space from which a portion of the intervertebral disk 20 was removed and W is maximized to provide the desired stabilization to adjacent vertebrae 16 and 18. Third and fourth sides 34 are arched from one end of implant 22 to the other to provide the portion of implant 22 intermediate the ends 25 and 36 with a width W which is larger than the width W' and W" at the 25 and 36, respectively. By comparison of the widths at the ends and middle portions of implant 22, it can be seen that in the embodiment shown in Fig. 3, the width W' at the end 25 of implant 22 is less than the width W" at the end 36 of implant 22. Because the sides 32 of implant 22 are substantially flat and the sides 34 are arched from one end 25 to the other end 36, implant 22 is described as being a bi-planar, bi-convex implant.

In the embodiment shown in Figs. 1-3, both the implant 22 and locking piece 24 are comprised of bone that is harvested and fabricated in the manner described below. Those skilled in the art who have the benefit of this disclosure will recognize that implant 22 and locking piece 24 may also be comprised of materials such as metal or polymeric materials so long as the material comprising implant 22 and locking piece 24 is biologically inert and/or minimally objectionable to the body's normal physiological processes. Although not shown, in one embodiment, the bi-convex sides 34 of implant 22 are provided with a plurality of teeth for biting into the adjacent vertebrae 16 and 18. So as to provide additional resistance to anterior-posterior (forward or backward) movement of implant 22 in the disk space, the teeth located closest to the end 25 of implant 22 (e.g., the teeth in the distal portion of implant 22) may be oriented at a slant toward the end 25 and the teeth closest to the end 36 of implant 22 may be oriented at a slant toward the end 36. The teeth in the middle portion of implant 22, e.g., between the two sets of slanted teeth, are then oriented vertically. It will also be recognized that the sides 34 of implant 22 need not be provided with the sensations or teeth to bite into the adjacent vertebrae. This biting function can also be accomplished by providing the sides 34 with multiple steps formed in right angles from the narrowest portions at the ends 25 and 36 to the widest portion in the approximate middle of implant 22 (i.e., from the dimension W' to W to W"). Alternatively, because in the embodiment shown the implant 22 is comprised of bone (see below), the surfaces of the sides 34 of implant 22 are scored, grooved, or even just "roughened up" either by the person fabricating the implant or by the surgeon at the time of implantation. Locking piece 24 can also be provided with teeth, serrations, or other structure to decrease the likelihood of extrusion of locking piece 24 from the disk space.

Those skilled in the art who have the benefit of this disclosure will recognize from the preceding paragraph that the sides 34 of implant 22 need not define an arch which is symmetrical from the end 25 to the end 36 of implant 22. Regardless, the end 25 of implant 22 is formed in a blunt, or rounded shape to reduce the likelihood of injury to the nerves of the spinal cord during insertion into the disk space.

In the embodiment shown, locking piece 24 is substantially square when viewed from the end 40 along the longitudinal axis 28. The sides of the square end 40 of locking piece 24 comprise the surfaces 50 for bearing against the bodies 12 and 14 of adjacent vertebrae 16 and 18 as also explained in more detail below. It will be recognized by those skilled in the art who have the benefit of this disclosure that the bearing surfaces 50 need not be flat and that the end 40 of locking piece 24 need not be square. Other shapes and configurations may be utilized as needed to insure that movement of locking piece 24 is resisted by the bodies of the adjacent vertebrae 16 and 18. Best results are obtained, however, when at least the vertebral bearing surface is oriented at an angle of approximately 90° to the implant bearing surface. The surfaces 42 of the locking piece 24 are substantially flat for contacting the first and second sides 32 of implant 22 to prevent rotation of implant 22 relative to locking piece 24 when locking piece 24 and implant 22 inserted into the disk space.

The purpose of the bi-planar; middle expanded, bi-convex implant 22 is to enable insertion of the implant 22 into the disk space and turning by approximately 90° to increase the disk height and stabilize the disk space. The purpose of locking piece 24 is to lock implant 22 against instability when in the vertical position, e.g., with the sides 32 parallel to the axis of the patient's spinal column, so as to maintain the disk height thereafter.

The sides 32 of implant 22 are provided with a keyway 46. Referring now to Fig. 3, an applicator (for use in connection with the present invention) is shown at 152 and is provided with an end 154 shaped in the form of a pair of prongs 144. The prongs 144 are formed in a size and shape substantially complementary to the size and shape of the keyway 46 of implant 22. Applicator 152 is mounted to implant 22 by inserting the prongs 144 into the keyways 46 formed on the sides 32 of implant 22. In this manner, the prongs seat the implant 22 on the end 154 of applicator 152 and resist relative rotational movement between implant 22 and applicator 152 when applicator 152 is used to rotate implant 22 in the disk space.

Although not shown in the figure, those skilled in the art who have the benefit of this disclosure will recognize that the end of the keyways 46 may be extended along the sides 32 of implant 22 further than is necessary to receive the prongs 144 on applicator 152 and that the extra length of the keyways 46 may be of gradually reducing dimension so that the prongs 144 of applicator 152 are received in a friction fit in the keyways 46 to help affirmatively mount implant 22 thereto. Other structure for achieving this same result includes a detent or serrations formed in the keyways 46. The mouth 45 of the keyway 46 on the side 32 of implant 22 at the first end 36 of the implant is wider than the width of the keyway 46 in the portion of the slot intermediate the ends 25 and 3 6 of implant 22 to facilatate insertion of the prongs 144 of applicator 152 into the keyway 46. The funnel-shaped portion 47 of the keyway 46 behind the mouth 45, which gradually decreases in width, acts to increase the ease with which implant 22 is mounted to applicator 152 by insertion of the prongs 144 into the respective keyways 46 and helps to seat implant 22 thereon. Those skilled in the art who have the benefit of this disclosure will recognize that the keyway 46 may be located on the applicator 152 and a key 48 may be located on implant 22 without any difference in the manner in which those component parts function to retain implant 22 on the end 154 of applicator 152.

When the end 154 of applicator 152 is seated all the way into the keyways 46 of implant 22, so as to prevent relative rotational movement therebetween, implant 22 is inserted into the disk space and rotated therein using applicator 152 as explained below. Applicator 152 is then detached from implant 22 simply by withdrawing the applicator 152 from the disk space, the friction exerted by the adjacent vertebrae preventing the withdrawal of the implant 22. It will be apparent to those skilled in the art who have the benefit of this disclosure that the applicator 152 is of little assistance in removing the implant 22 from the disk space even if the keyways 46 of implant 22 are provided with a detent or other structure to engage the prongs 144 of applicator 152 to retain the implant 22 thereon. As noted above, in the embodiment described herein, implant 22 is comprised of bone. In alternative embodiments, implant 22 is comprised of metal or inert polymeric material and, when comprised of such material, the implant may also include a bore (not shown) in the end 36 of implant 22 for receiving a complementary threaded end (not shown) on applicator 152. The advantage to using such materials is that, in the event the implant 22 needs to be removed from the disk space, an applicator of the type shown in Patent No. 5,658,336, is screwed into the bore to allow the implant 22 to be pulled from the disk space. If implant 22 is comprised of bone as contemplated herein and it is necessary to remove the implant 22, the implant is simply drilled out with a conventional bone drill or burr and the pieces drawn back out of the disk space with forceps or other suitable instrument to be replaced with another implant as needed.

The use of the stabilizer 10 in, for instance, a method of lumbar intervertebral disk stabilization is illustrated in Fig. 1. Surgery is performed as in a simple diskectomy and the intervertebral disk 20 is exposed through a small laminotomy. The disk material is removed and any nerve root compression is corrected. The posterior longitudinal ligament (not shown) and disk cartilage are removed until the surface of the bodies 12 and 14 of adjacent vertebrae 16 and 18, respectively, are exposed above and below the disk space.

Using spreaders such as those disclosed in International Application No. PCT/US95/00347, the vertebrae 16 and 18 are distracted to open the disk space, and once the desired "spread" is achieved, the middle portion of the disk space is packed with cancellous bone chips. Because the posterior longitudinal ligament is left intact to the opposite side and to the center of the disk space, the bone chips are held in place in the disk space.

An implant 22 having a height H and width W selected to fit the disk space is then mounted to the prongs 144 of applicator 152. The appropriately-sized implant 22 is then inserted into the disk space using the applicator 152 with the implant 22 oriented so that the top and bottom thereof, i.e., the first and second sides 32, engage the bodies 12 and 14 of adjacent vertebrae 16 and 18, respectively. Using the applicator 152, implant 22 is positioned in the disk space by anterior-posterior movement to a position in which the expanded, middle portion and the smaller width ends 25 and 36 of the third and fourth sides 34 of implant 22 contact the respective lower and upper surfaces of the bodies 12 and 14 of the adjacent vertebrae 16 and 18 when rotated by approximately 90° using the applicator 152. The respective lower and upper surfaces of the vertebral bodies 12 and 14 are slightly concave such that the larger width middle portion W" of implant 22 allows the implant 22 to engage substantially more of the respective surfaces of the vertebral bodies 12 and 14 than conventional prosthetic devices, thereby providing increased stability to the fusion once further rotation of implant 22 in the disk space is prevented as described below.

Once positioned in the disk space so as to provide maximum stabilization, the applicator 152 is then detached from the implant 22 by backing out of the incision in the patient. Locking piece 24 is then inserted through that same incision and pressed or impacted into place in the disk space adjacent and lateral to implant 22 with the implant bearing surface 42 of locking piece 24 juxtaposed and/or engaging the surface 32 of implant 22. Positioning the locking piece 24 adjacent implant 22 in this manner resists relative rotation between implant 22 and locking piece 24. Because the bearing surfaces 50 of locking piece 24 bear against the bodies 12 and 14 of the adjacent vertebrae 16 and 18 to resist rotation of the locking piece 24 relative to the adjacent vertebrae 16 and 18 against which the bearing surfaces 50 bear, rotation of the implant 22 is also resisted. Those skilled in the art who have the benefit of this disclosure will recognize that the bearing surfaces 50 bear against the cortical end plate of the respective vertebral bodies 12 and 14, which is comprised of cortical, non-cancellous bone, and provides a hard, relatively smooth surface against which the bearing surfaces 50 bear. The end 40 of locking piece 24, which is also preferably comprised of bone, may then be shaped to different sizes and shapes (other than the square shaped end 40 shown in the figures) so as to allow the surgeon to select an appropriately size and shape that provides a close fit with the space between vertebral bodies.

If necessary, a small amount of a physiologically compatible adhesive of a type known in the art is applied over the cancellous bone chips just medial to the implant to close off the remaining portion of the opening into the disk space. The patient should be able to ambulate soon after the procedure because of the stability imparted to the spinal column by the implant. Before narrowing of the disk space occurs, the cancellous bone chips will have started the fusion process.

The stabilizer 10 is also used to advantage to perform, for instance a posterior lateral intertransverse fusion. The implant 22 is inserted into the region of the disk space from which a portion of the disk has been removed as described above with the locking piece 24 inserted and the posterior lateral fusion performed. Because the implant 22 provides stability to the spine until the posterior lateral fusion is solid, the patient is generally able to ambulate soon after surgery. This procedure also prevents the narrowing of the disk space, which is a common problem with posterior lateral fusion.

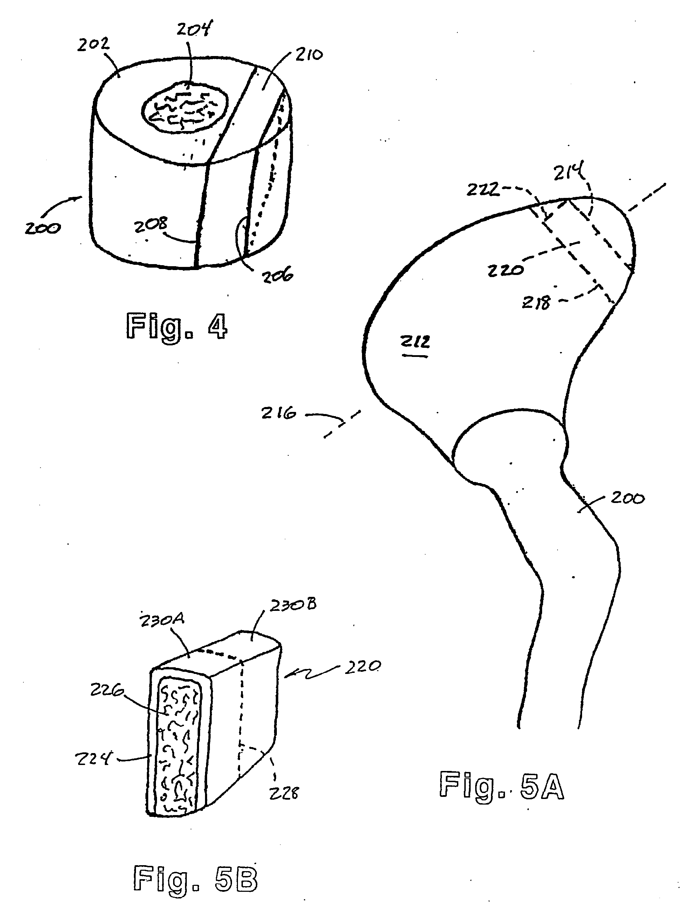

Referring now to Fig. 4, one preferred method for fabricating the implant 22 will now be described. Fig. 4 shows a perspective view of a cross-sectioned portion of the shaft of the human femur 200. Femur 200 is comprised of hard cortical bone 202 surrounding a central core of softer, cancellous bone 204. Implant 22 is fabricated from the cortical bone 202 of the piece of femur 200 by making a first vertical cut along the longitudinal axis of the femur 200 in the area of the cortical bone 202 as shown at reference numeral 206 and a second vertical cut along the longitudinal axis of the femur 200 in the cortical bone 202, closer to the cancellous bone 204 in the center axis of femur 200 along the line indicated at 208. The resulting piece of the cortical bone 202 comprising the femur 200, identified by reference numeral 210 in Fig. 4 serves as a '"blank" for making the implant 22. Using conventional bone saws and shaping techniques, the blank 210 is shaped to include the bi-convex first and second sides 32, rounded and 36, and so on as described above. Although the lines 206 and 208 representing the first and second vertical cuts are shown substantially parallel to each other, those skilled in the art who have the benefit of this disclosure will recognize that lines 206 and 208 need not be parallel and that there is even certain advantages to be gained by not making the cuts parallel as, for instance, when it is desired to make an implant that is wedge-shaped as described below. Those skilled in the art who have the benefit of this disclosure will also recognize that there are other bones in the body from which a piece of cortical bone can be harvested that is big enough to serve the same role as the blank 210, the femur 200 being described herein merely for the purpose of enabling the present invention as required by the Patent Statute.

Referring now to Figs. 5A and 5B, Fig. 5A is a schematic representation of the human iliac 212 and femur 200 in the area of the hip. In the presently described embodiment, locking piece 24 is fabricated from a portion of the iliac 212. Implant 22 is fabricated from the hard cortical bone 202 of the femur 200 to give the implant 22 the strength to properly support the load of the spinal column. By contrast, because it is desired that the iliac 212 have properties that both impart strength to locking piece 24 and facilitate its fusion with the cancellous bone chips packed around the implant 22 in the disk space as well as the adjacent vertebral bodies 16 and 18, the locking piece 24 is preferably fabricated from a combination of cortical and cancellous bone and that combination is found in the iliac 212. Specifically, a first cut is made that is orthogonal to the long axis of the iliac (the latter being represented by the shadow line 216 in Fig. 5A) in the so-called "wing" of the iliac 212 as shown at line 214. A second orthogonal cut 218, substantially parallel to the first cut 214, is then made across the wing of the iliac 212 to produce a piece of bone 220 that is in effect a cross-sectional piece of the iliac. The piece of bone 220 is then squared up at one end by cutting along the line 222. Because the cuts 214, 218, and 222 can be made in any order such that the piece of bone 220 may not be removed from the iliac 212 when cut 222 is made, cut 222 is described herein as being substantially parallel to the long axis 216 of the iliac 212.

The piece of bone 220 is shown removed from the iliac 212 and in perspective view in Fig. 5B. When viewed in this manner, it will be apparent that the piece 220 of bone harvested from the iliac 212 in the above-described manner is comprised of both hard cortical bone as shown at reference numeral 224 and softer cancerous bone as shown at 226. To make two of the locking pieces 24, a cut 228 is made through the piece of bone 220. Again, it will be recognized by those skilled in the art that the cut 228 can be made in any order relative to the cuts 214, 218, and 222 such that the cut 228 can be described as being substantially parallel to the cuts 214 and 218 and orthagonal to the longitudinal axis 216 of the iliac 212. The cortical bone 224 forms a relatively thin outer layer around the two sides and one end of each of the stabilizers 230A and 230B that are fabricated from piece 220, such that the stabilizer of the present invention is referred to as being "tri-cortical," and will form the implant bearing surface 50 and end 40 of stabilizer 24 as shown in Figs. 1-3. The central core of cancellous bone 226 promotes the fusion of the locking piece 24 in the disk space as described above, but is not a necessary element of the invention, it being apparent from this disclosure that a locking piece can be fabricated entirely from cortical bone or cancellous bone, the tri-cortical locking piece described herein being only a preferred embodiment of the locking piece. Those skilled in the art who have the benefit of this disclosure will also recognize that there are other bones in the body from which a piece of cortical bone can be harvested that is big enough to serve the same role as the piece 220.

In the embodiments described herein, the implant 22 and locking piece 24 are fabricated to certain preferred dimensions, but those skilled in the art will recognize that it is desirable to fabricate them in several dimensions so as to facilitate a close fit in the disk space. For instance, a presently preferred size for implant 22 is for implant 22 to have a height H of approximately 5 mm, a width W of about 10 mm at the ends 25, 36 increasing to a maximum of about 15 mm, and a length (along the longitudinal axis of implant 22) of about 27 mm. A locking Piece 24 intended fur use with an implant of this 15 X 5 X 27 size is about 10 mm X 10 mm X 27 mm. Other implant, and corresponding locking piece, sizes are also contemplated, it being understood that because they are comprised of bone in this preferred embodiment, the bone can be harvested in the desired shape and size from several locations of the skeleton.

Referring now to Fig. 6A, another embodiment of an implant which is an implant constructed in accordance with the present invention is shown. Specifically, the implant 322A is substantially rectangularly shaped in cross-section and, like implant 22 in Figs. 1 - 3, is comprised of first and second sides 332 defining the height H and third and fourth sides 334 defining width W, the height dimension H being greater than the width dimension W. Third and fourth sides 334 are arched from one end of implant 322 to the other to provide the portion of implant 322 intermediate the ends 325 and 336 with a width W that is larger than the width W' and W" at the ends 325 and 336, except that the end 325 is flared as at 360 to provide the implant 322A with a width W' at the end 325 that is approximately equal to the width of implant 322A at the maximum width W. The two sides 334 therefore comprise two opposed edges, each of arched form, the opposed edges being shaped such that the end 325 is flared so that a portion of the implant 322A between the flared end part 360 and the remainder of the implant is of lesser width than the width W' of the flare 360 in the end 325 of implant 322A. This portion of lesser width of implant 322A forms upwardly and downwardly opening recesses 362 and is preferably formed as a smoothly curved recess to interact with and provide stability for the adjacent vertebrae, the articulating surfaces of which are concave in shape. As noted above in connection with the description of the embodiment shown in Figs. 1-3, implants constructed in accordance with the teachings of the present invention may be provided with means for resisting anterior-posterior movement of the implant in the disk space. In the embodiment shown in Fig. 6, implant 322 is provided with a plurality of teeth 338 for this purpose. As also noted above, the teeth near the ends of the implant may be slanted toward the ends of the implant so as to provide better contact with the adjacent vertebrae, but in the case of the implant 322 shown in Fig. 6, it has been found that better contact is obtained by slanting the teeth in the flared portion 360 away from the end 325.

As shown in Figs. 6B and 6C, the width W' of the ends 325 of implants 322B and 322C is progressively greater than the width of implants 322B and 322C at the approximate midpoint along the longitudinal axes of implants 322B and 322C shown as the maximim width W. Stated another way, the difference in the widths W and W' of implants 322B and 322C is defined by the angle α formed by extending lines from the midpoint of the implant 322B and 322C representing width W to the tip of the flare 360 at the end 325 of each respective implant representing width W. It is envisioned that several implants 322 would be provided with different angles α to accommodate specific patient needs; for instance, in one patient, the curvature of the spine, or lordosis, may require that an implant having an angle α that provides a width W' that is 5 mm larger than the width W be utilized between two vertebrae and that a second implant having an angle α that provides a width W' that is 10 mm larger than the width W may be needed between the next two vertebrae to maintain the proper lordosis of the spine. Of course the angle α can be any angle, but it is generally preferred that an angle α of between 0° (those skilled in the art will recognize that the angle α of the embodiment shown in Fig. 6A is 0°) and 30° will provide a sufficient range of width W' dimensions to accommodate most patient needs.

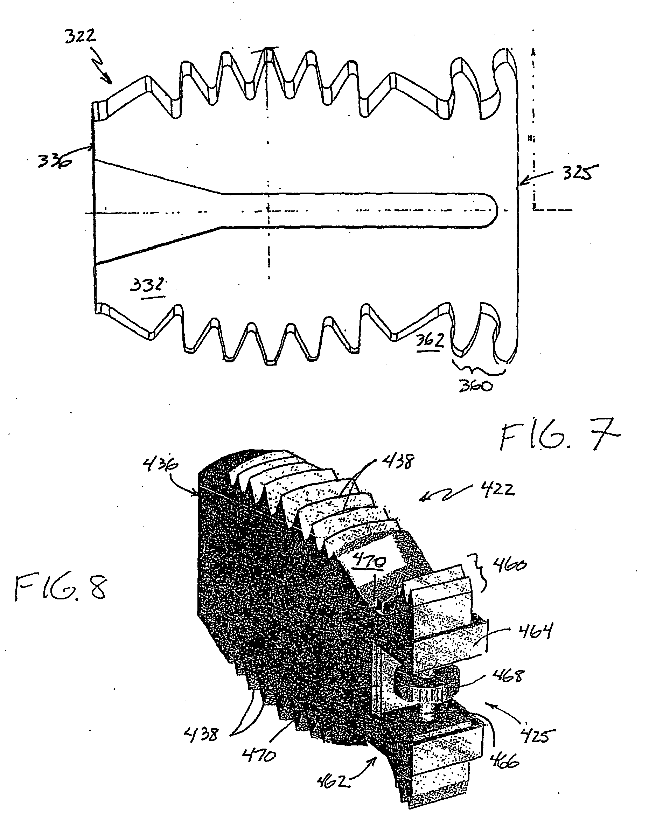

Figure 7 shows an alternative embodiment of the implant 322 shown in Fig. 6 wherein the sides 332 of the implant 322 are provided with keyways 46 having a funnel-shaped mouth 47 for receiving the prongs 144 of applicator 152 as described above. In Fig. 7, the implant 322 is provided with an angle α of 0° such that the dimensions W and W' are approximately equal, but those skilled in the art will recognize that the implant shown in Fig. 7 could have been formed with a flared end such that the angle α could have been any angle from about 0° to about 30°.

If the alternative embodiment shown in Fig. 8, is utilized, the surgeon need not select an implant having a desired angle α. Instead, the implant 422 shown in Fig. 8 is provided with a pair of blocks 464 movable relative to the implant in a direction having a component that is perpendicular to the longitudinal axis of implant 422 for changing the width W' of the flare 460 at the end 425 of implant 422. Blocks 464 are threadably engaged to a spindle 466 that is journaled in implant 422 and means is provided, in the form of the wheel 468 integral with spindle 466, for rotating the spindle 466 to spread the blocks 464 apart, or to move the blocks 464 closer to the mid-line, or longitudinal axis, of implant 422. A track 470 is formed in implant 422, and the surface of the blocks 464 adjacent the track 470 is formed in a complementary shape (not shown) for engaging the track 470 to define the direction of in which the blocks 464 slide along track 470 relative to implant 422. Those skilled in the art who have the benefit of this disclosure will recognize that the direction of movement of blocks 464 relative to implant 422 need not be perpendicular to the longitudinal axis of implant 422 and that it may even be advantageous to move blocks 464 in a direction defined by an angle that is not a 90° angle relative to the longitudinal axis of implant 422. For this reason, the movement of the blocks 464 is described herein as being in a direction "having a component that is perpendicular" to the longitudinal axis of implant 422 rather than in a direction that is perpendicular to the longitudinal axis of implant 422.

Although described in terms of the preferred embodiments shown in figures 6 to 8, it will be recognized by those skilled in the art that certain changes can be made to the specific structure of the preferred embodiments shown and described without departing from the present invention. In the case of one such change, the first and second sides of the implant are substantially flat but not parallel along their longitudinal axes so that the implant is wedge-shaped. The wedge shape of the implant facilitates insertion of the implant into the disk space, the rounded end of the implant reducing the likelihood of injury to the nerves of the spinal cord during insertion into the disk space. Likewise, the width at one end of the implant can be less than the width at the end, both widths, however, being less than the width in the middle, expanded portion of the implant. All such modifications, and other modifications which do not depart from the present invention, are intended to fall within the scope of the following claims.

## Claims

1. An apparatus for use in fusing adjacent vertebrae, the apparatus comprising an elongate implant (322A) to be located between adjacent vertebrae, the implant being of elongate form and having two opposed sides (334) which define the width W of the implant, each of the two opposed sides being of a side edge of arched form, **characterised in that** the opposed edges (334) are such that an end part (325) of the implant is flared so that a portion of the implant between the flared end part (325) and the remainder of the implant is of lesser width that the width of the flare.

2. An apparatus according to Claim 1 wherein the arched edges (334) and flare (335) form an upwardly open recess (362) in the top part of the Implant (332A) and a downwardly open recess (362) in the bottom part of the implant, the recesses being smoothly curved recesses.

3. An apparatus according to Claim 2 wherein the recesses (362) are substantially symmetrical about a horizontal plane dividing the upper and lower parts of the implant (322A).

4. An apparatus according to any one of Claims 1-3 wherein the flared end part (460) of the implant (422) is constituted by at least one block (464) which is movably mounted on the rest of the implant for changing the width of the implant at the flared end part thereof.

5. In apparatus according to Claim 4 wherein there are two said blocks (460), each slidably mounted to the rest of the implant (422).

6. An apparatus according to Claim 2 or 4 wherein each of said blocks (460) is threadably engaged with a rotatable spindle (466), the spindle being mounted on the rest of the implant, means (468) being provided to rotate the spindle.

7. An apparatus according to any one of the preceding Claims wherein the implant (322A) has two opposed substantially planar faces.

8. An apparatus according to Claim 7 wherein key-ways (46) are formed in the planar faces and extend axially of the implant.

9. An apparatus according to Claim 8 wherein each key-way (46) is provided with a funnel shaped terminal portion (47), the funnel shaped portion converging to a width approximately equal to the width of the main part of the key-way.

10. An apparatus according to any one of Claims 8-10 further comprising an applicator (152) having prongs (144) adapted to co-operate with said key-ways (46).

## Patentansprüche

1. Vorrichtung zur Verwendung bei der Fusion von benachbarten Wirbeln, wobei die Vorrichtung ein längliches Implantat (322A) umfaßt, das zwischen benachbarten Wirbeln anzuordnen ist, wobei das Implantat eine längliche Form aufweist und zwei gegenüberliegende Seiten (334) aufweist, die die Breite W des Implantats bilden, wobei jede der beiden gegenüberliegenden Seiten eine Seitenkante mit Bogenform aufweisen, **dadurch gekennzeichnet, daß** die gegenüberliegenden Kanten (334) derart sind, daß ein Endteil (325) des Implantats so nach außen erweitert ist, daß ein Abschnitt des Implantats zwischen dem nach außen erweiterten Endteil (325) und dem Rest des Implantats eine geringere Breite als die Breite der Erweiterung aufweist.

2. Vorrichtung nach Anspruch 1, **dadurch gekennzeichnet, daß** die gebogenen Kanten (334) und Erweiterung (335) eine nach oben offene Aussparung (362) in dem oberen Teil des Implantats (332A) und eine nach unten offene Aussparung (362) in dem unteren Teil des Implantats bilden, wobei die Aussparungen sanft gekrümmte Aussparungen sind.

3. Vorrichtung nach Anspruch 2, **dadurch gekennzeichnet, daß** die Aussparungen (62) im wesentlichen symmetrisch um eine horizontale Ebene sind, die die oberen und unteren Teile des Implantats (322A) unterteilt.

4. Vorrichtung nach einem der Ansprüche 1-3, **dadurch gekennzeichnet, daß** der nach außen erweiterte Endteil (460) des Implantats (422) durch mindestens einen Block (464) gebildet ist, der an dem Rest des Implantats zur Änderung der Breite des Implantats an dessem nach außen erweiterten Endteil bewegbar montiert ist.

5. Vorrichtung nach Anspruch 4, **dadurch gekennzeichnet, daß** zwei genannte Blöcke (460) vorhanden sind, wobei jeder an dem Rest des Implantats (422) verschiebbar montiert ist.

6. Vorrichtung nach Anspruch 2 oder 4, **dadurch gekennzeichnet, daß** jeder der Blöcke (460) mit einer drehbaren Spindel (466) in Schraubeingriff steht, wobei die Spindel an dem Rest des Implantats montiert ist und ein Mittel (468) zum Drehen der Spindel vorgesehen ist.

7. Vorrichtung nach einem der vorangehenden Ansprüche, **dadurch gekennzeichnet, daß** das Implantat (322A) zwei gegenüberliegende im wesentlichen planare Flächen aufweist.

8. Vorrichtung nach Anspruch 7, **dadurch gekennzeichnet, daß** Keilnuten (46) in den planaren Flächen ausgebildet sind und sich axial vom Implantat erstrecken.

9. Vorrichtung nach Anspruch 8, **dadurch gekennzeichnet, daß** jede Keilnut (46) mit einem trichterförmigen Einführungsabschnitt (47) versehen ist, wobei der trichterförmige Abschnitt auf eine Breite zusammenläuft, die näherungsweise gleich der Breite des Hauptteils der Keilnut ist.

10. Vorrichtung nach einem der Ansprüche 8-10, **dadurch gekennzeichnet, daß** sie ferner einen Applikator (152) mit Zinken (144) umfaßt, die gestaltet sind, um mit den Keilnuten (46) zusammenzuwirken. -

## Revendications

1. Appareil destiné à être utilisé pour fusionner des vertèbres adjacentes, l'appareil comprenant un implant allongé (322A) à positionner entre des vertèbres adjacentes, l'implant étant de forme allongée et possédant deux côtés opposés (334) qui définissent la largeur W de l'implant, chacun des deux côtés opposés étant de bord latéral en forme d'arc, **caractérisé en ce que** les bords opposés (334) sont tels qu'une partie d'extrémité (325) de l'implant est évasée de telle façon qu'une portion de l'implant entre la partie d'extrémité évasée (325) et le reste de l'implant est de largeur moindre que la largeur de l'évasement.

2. Appareil selon la revendication 1 dans lequel les bords arqués (334) et l'évasement (335) forment un retrait ouvert vers le haut (362) dans la partie supérieure de l'implant (332A) et un retrait ouvert vers le bas (362) dans la partie inférieure de l'implant, les retraits étant des retraits à courbure douce.

3. Appareil selon la revendication 2 dans lequel les retraits (362) sont substantiellement symétriques par rapport à un plan horizontal divisant les parties supérieure et inférieure de l'implant (322A).

4. Appareil selon l'une quelconque des revendications 1 à 3 dans lequel la partie d'extrémité évasée (460) de l'implant (422) est constituée par au moins un bloc (464) qui est monté de façon mobile sur le reste de l'implant afin de modifier la largeur de l'implant au niveau de la partie d'extrémité évasée de celui-ci.

5. Appareil selon la revendication 4 dans lequel il y a deux desdits blocs (460), chacun monté de façon coulissante par rapport au reste de l'implant (422).

6. Appareil selon la revendication 2 ou 4 dans lequel chacun desdits blocs (460) est mis en prise par vissage avec une broche apte à tourner (466), la broche étant montée sur le reste de l'implant, des moyens (468) étant fournis pour faire tourner la broche.
